# EUROPÄISCHE PATENTSCHRIFT

(11) **EP 0 540 560 B1**
(45) Veröffentlichungstag und Bekanntmachung des Hinweises auf die Patenterteilung: **29.11.1995**
(21) Anmeldenummer: 91912909.8
(22) Anmeldetag: 19.07.1991
(51) Int. Cl.: C12N 15/57, C12N 9/64, A61K 38/46

(54) **ANCROD ÄHNLICHE PROTEINE, IHRE HERSTELLUNG UND VERWENDUNG**
ANCROD-LIKE PROTEINS, THEIR PREPARATION AND THEIR USE
PROTEINES SEMBLABLES A L'ANCROD, LEUR FABRICATION ET UTILISATION

(30) Priorität: 26.07.1990 DE 4023699
(43) Veröffentlichungstag der Anmeldung: 12.05.1993
(73) Patentinhaber: BASF Aktiengesellschaft, 67063 Ludwigshafen (DE)
(72) Erfinder: BACH, Alfred, D-6802 Ladenburg (DE); HILLEN, Heinz, D-6802 Ladenburg (DE); BIALOJAN, Siegfried, D-6836 Oftersheim (DE)
(86) Internationale Anmeldenummer: EP9101361
(87) Internationale Veröffentlichungsnummer: WO9201794

(56) Entgegenhaltungen:
- EP-A- 0 395 375
- WO-A-90/06362
- US-A- 4 585 653
- US-A- 4 610 879

## Beschreibung

Die vorliegende Erfindung betrifft neue Proteine mit fibrinogenolytischen Eigenschaften, sog. Fibrinogenasen, deren Herstellung und Verwendung zur Prophylaxe und Therapie von Krankheiten.

Aus dem Gift der malaiischen Grubenotter (Agkistrodon rhodostoma) konnte bislang nur ein Fibrinogen-spaltendes Enzym gewonnen werden, welches antikoagulatorische Eigenschaften besitzt (Biochem, J. 131, 799, 1973). Dieses Protein wird in der Literatur als Arvin, Arwin oder Ancrod bezeichnet.

Die Anwendungsmöglichkeiten dieses Proteins sind begrenzt, da nach 6 bis 8 Wochen Resistenzerscheinungen auftreten können, die vermutlich auf die Bildung von Ancrod-neutralisierenden Antikörpern zurückzuführen sind. Vereinzelt kommt es auch zu hämorrhagischen Komplikationen.

Es wurden nun weitere Proteine mit fibrinogenolytischen Eigenschaften gefunden und rein hergestellt.

Gegenstand der Erfindung sind glykosylierte, partiell glykosylierte oder nicht glykosylierte Polypeptide mit den im Sequenzprotokoll angegebenen Aminosäuresequenzen 1, 2, 3, 4 und 5, worin Xaa und Xab Reste natürlicher α-Aminosäuren darstellen, sowie deren Allelvarianten, die mit den angegebenen Sequenzen in mehr als 95 % der Aminosäureprositionen identisch sind.

Der Aminosäurerest Xaa stellt Asn, Gln, Ser, Thr, Gly, Asp, Glu, Lys, Arg oder Pro, vorzugsweise jedoch Asn, Gln, Ser und Thr und insbesondere Asn und Gln dar. Xab ist vorzugsweise Phe, Tyr, Leu, Ile, Ala, Val, Thr oder Ser.

Die Erfindung betrifft weiter DNA-Sequenzen, die für die oben genannten Proteine kodieren, sowie Vektoren, die diese DNA-Sequenzen enthalten. Bevorzugte DNA-Sequenzen sind im Sequenzprotokoll, Sequenz Nr. 6 bis 9, wiedergegeben.

Die erfindungsgemäßen Proteine lassen sich gentechnisch nach bekannten Methoden herstellen.

So kann man aus dem Drüsengewebe einer malaiischen Grubenotter (Agkistrodon rhodostoma) mRNA isolieren und in doppelsträngige cDNA übersetzen. Nach Einsetzen dieser cDNA in einen kommerziell erhältlichen Klonierungsvektor z.B. λ gt 10 wird eine cDNA-Bibliothek angelegt. Die dabei verwendeten Methoden sind beispielsweise in Maniatis et al., Molecular Cloning, CSH Press, (1982) nachzulesen. Auch das Screening solcher Genbanken mit radioaktiv markierten Oligonukleotidsonden oder radioaktiv markierten DNA-Fragmenten ist inzwischen eine vielfach verwendete und beschriebene Methode. Nach diesem Verfahren kann ein cDNA-Klon, der Homologie zur Oligonukleotidsonde oder zu radioaktiv markierten DNA-Fragmenten besitzt isoliert und charakterisiert werden. Dieses Verfahren ist in "DNA cloning Vol I, IRL Press, 1985" beschrieben.

Die so charakterisierte cDNA ist mit Hilfe von Restriktionsenzymen leicht zugänglich. Die dabei entstehenden Fragmente, ggf. in Verbindung mit chemisch synthetisierten Oligonukleotiden, Adaptoren oder Genfragmenten, können benutzt werden, um die für das Protein kodierende Sequenzen zu klonieren. Der Einbau der Genfragmente bzw. synthetischen DNA-Sequenzen in Klonierungsvektoren, z. B. die handelsüblichen Plasmide M13mp oder pkk-223-3, erfolgt in bekannter Weise. Auch können die Gene oder Genfragmente mit geeigneten chemisch synthetisierten oder aus Bakterien, Phagen, Eukaryontenzellen oder deren Viren isolierten Kontrollregionen versehen werden, die die Expression der Proteine ermöglichen.

Die Transformation bzw. Transfektion geeigneter Wirtsorganismen mit den so erhaltenen Hybridplasmiden ist ebenfalls bekannt und eingehend beschrieben (M. Wigler et al., Cell 16 (1979) 777-785; F.L. Graham and A.J. van der Eb, Virology 52 (1973) 456-467). Auch können die Hybridplasmide mit entsprechenden Signalsequenzen versehen werden, die die Sekretion der Polypeptide ins Medium erlauben.

Bei der Expression in Säugerzellen kann man Vektoren verwenden, die das zu exprimierende Gen, in diesem Fall die cDNA, welche für eine der im Sequenzprotokoll beschriebenen Fibrinogenasen kodiert, unter die Kontrolle des Maus-Metallothionein- oder des viralen SV40-Promotors setzt (J. Page Martin, Gene, 37 (1985) 139-144). Notwendig für die Expression ist das Vorliegen des Methionin-Startcodons und der Leader-/Prosequenz des Gens für das entsprechende Protein. Man isoliert dann Klone, die Kopien dieser Vektoren als Episome oder ins Genom integriert besitzen. Besonders vorteilhaft sind die Integration und Expression des Fremdgens auf der Basis des Rinderpapillom-Virus. In Verbindung mit prokaryontischen Sequenzen, die für die Replikation in Bakterienzellen und eine Antibiotikum-Resistenz kodieren, ist der Aufbau von "shuttle"-Vektoren möglich. Konstruktion und Vermehrung des Plasmids erfolgen zunächst in Bakterienzellen; anschließend erfolgt die Umsetzung in die Eukaryontenzellen, z.B. in die Maus-Fibroblastenzellinie c127.

Auch andere Zellsysteme, z.B. Hefe und andere Pilze, Insektenzellen sowie tierische und humane Zellen wie z.B. CHO-, COS, L-und 293 Zellen, können in Verbindung mit geeigneten Expressionsvektoren zur Expression der klonierten cDNA verwendet werden.

Diese eukaryontischen Expressionssysteme besitzen den Vorteil, daß sie in der Lage sind, ihre Produkte effektiv und meist in nativer Form zu sezernieren. Ferner besitzen sie die Fähigkeit, ihre Produkte posttranslational zu modifizieren.

So erhalten die beschriebenen Fibrinogenasen bei der Expression in Eukaryontenzellen noch Glykosidseitenketten. Diese Seitenketten fehlen bei den in Bakterien hergestellten Polypeptiden. Die Glykosidseitenketten können auch enzymatisch mit Hilfe entsprechender Glykosidasen vollständig oder partiell entfernt werden. Die meisten in Bakterien exprimierten eukaryontischen Proteine, fallen in der Zelle als denaturierte Einschlußkörper an und müssen proteinchemisch renaturiert werden. Ferner sind Bakterien oft nicht in der Lage, die Initiatoraminosäure Methionin vom fertigen Protein abzuspalten. Durch die Verwendung von Sekretionssystemen können diese Schwierigkeiten umgangen werden (Donald Oliver, Ann. Rev. Microbiol. 39, 1985, 615-48; John Ghrayeb et al. The EMBO Journal 3, 1984, 2437-2442.

Aufgrund der Degeneration des genetischen Codes ist es aber auch möglich, andere DNA-Sequenzen, z.B. chemisch synthetisierte Gene mit unterschiedlicher DNA-Sequenz für die Expression der beschriebenen Fibrinogenasen zu benutzen. Mit Hilfe der klonierten Gene können durch etablierte Methoden der Mutagenese Varianten von diesen Fibrinogenasen mit ähnlicher Wirkung hergestellt werden.

Die Reinigung der erhaltenen Polypeptide erfolgt aus dem Kulturmedium durch chromatographische Verfahren, z.B. Affinitätschromatographie an Arginin-Sepharose®, Matrex-RedA-Sepharose®, Heparin-Sepharose® oder Ionenaustauschermaterialien in bekannter Weise (Lit.: "Guide to Protein Purification", Murray P. Deutscher [ed], Academic Press 1990).

Die Reinigung der Fibrinogenasen kann ebenfalls direkt aus dem Schlangengift der A. rhodostoma durch die Kombination geeigneter chromatographischer Verfahren, vorzugsweise unter Verwendung von Matrex-RedA-Sepharose®, Heparin-Sepharose®, Arginin-Sepharose®, ConA-Sepharose®, Q-Sepharose®, S-Sepharose® und Chromatofocussierung, wie im Beispiel 5 beschrieben, gereinigt werden. Für die Feinreinigung kommen insbesondere HPLC-Verfahren in Betracht.

Gegenstand der Erfindung sind auch Arzneimittel, welche die erfindungsgemäß hergestellten Proteine enthalten, ggf. in einem pharmazeutisch verträglichen Träger oder Bindemittel. Die Arzneimittel können auch Kombinationen der erfindungsgemäß hergestellten Proteine mit anderen pharmakologisch wirksamen Therapeutika enthalten, wie z.B. mit Thrombolytika (tPA, Streptokinase), Hirudin oder Thromboxanrezeptor-Antogonisten
Weitere Ausgestaltungen der Erfindung sind in den Beispielen näher beschrieben.

Für gentechnische Methoden sei dazu z.B. auf das Handbuch von Maniatis et al. "Molecular Cloning", Cold Spring Harbor Laboratory, 1982 oder "DNA cloning" Vol I - III, IRI Press 1985 - 87, Herausgeber D.M. Glover hingewiesen.

Die erfindungsgemäßen Polypeptide eignen sich zur Behandlung von Glomerulonephritis, Herzinfarkt, non-ischämischem Schlaganfall, peripheren arteriellen Durchblutungsstörungen (insbesondere Atherosclerosis obliterans, Thrombangitis obliterans, diabetische Mikroangiopathie und Morbus Raynaud), instabiler Angina pectoris, tiefer Venenthrombose und anderen Thrombosen, Rethrombosierung nach thrombolytischer Therapie, Rethrombosierung nach gefäßchirurgischen Eingriffen wie bei der Implantation arterieller oder venöser Gefäßplastiken sowie zur Vermeidung von Thrombosen im extrakorporalen Kreislauf.

### Beispiel 1

### Isolierung eines cDNA Klones für Fibrinogenasen aus der malaiischen Grubenotter (Agkistrodon rhodostoma)

1 g Giftdrüsengewebe einer 5 Jahre alten Schlange der Gattung Agkistrodon rhodostoma wurde in 6 M Guanidiniumthiocyanat, 5 mM Natriumcitrat (pH 7,0), 0,1 M 2-Mercaptoethanol, 0,5 % Sarcosyl im ULTRA-TURRAX® aufgeschlossen. Grobe Zelltrümmer wurden bei 3000 rpm abzentrifugiert. Die RNA wurde durch Zentrifugation durch ein 5,7 M CsCl-Kissen über Nacht bei 45.000 rpm abgetrennt. Anschließend wurde die polyA⁺-enthaltene RNA-Fraktion durch Affinitätschromatographie an oligo(dT)-Cellulose abgetrennt.

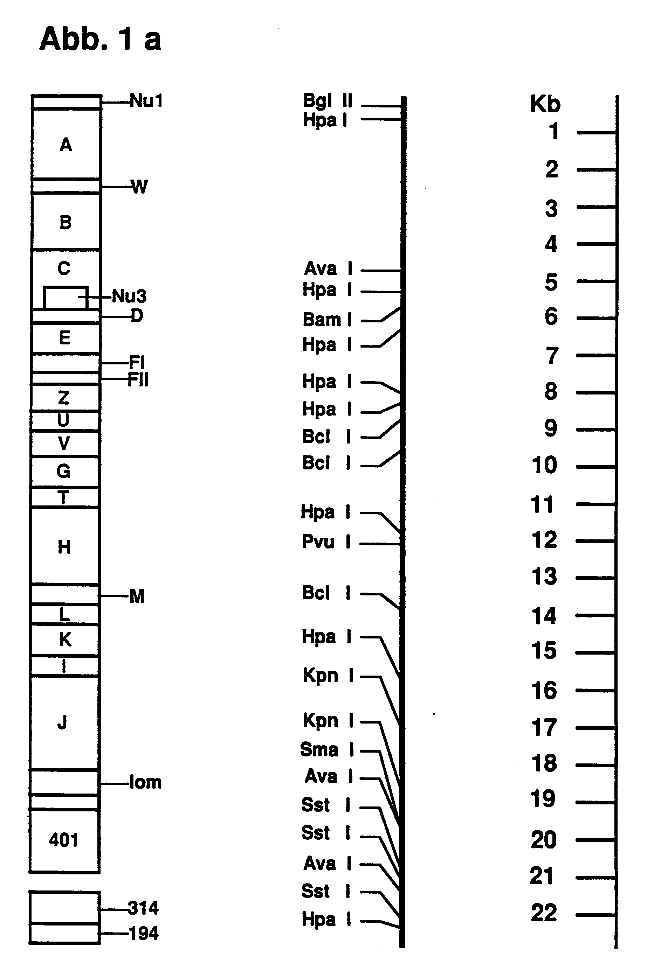

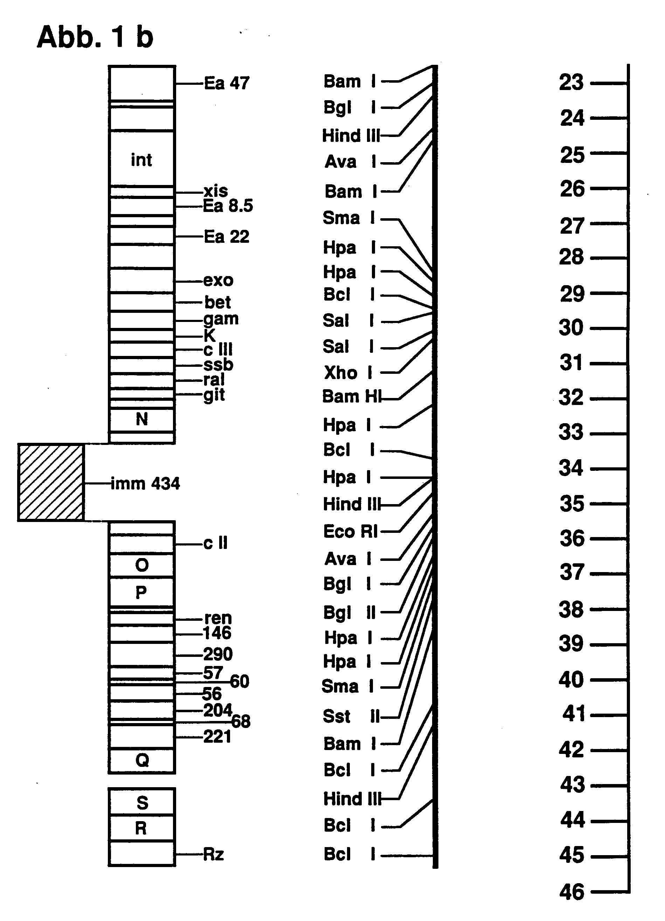

Mit Hilfe des Enzyms AMV-Reverse Transcriptase und oligo(dT)12-18 als Starter wurde die polyA⁺-RNA in einzelsträngige cDNA umgeschrieben. Die Synthese des zweiten Stranges erfolgte mit E.coli-DNA-Polymerase I. An die doppelsträngige cDNA wurde mit Hilfe des Enzyms T4-DNA-Ligase ein EcoRI Adaptor mit folgender Sequenz angesetzt: 5'AATT CCATGG ATG CATGC 3'. Der kommerziell erhältliche Phagenvektor λ gt 10 (Abb. 1a, 1b) wurde mit dem Restriktionsenzym EcoRI linearisiert. Beide DNAs wurden miteinander ligiert und mit dem kommerziell erhältlichen Verpackungsextrakt zu infektiösen Phagen verpackt. Die rekombinanten Phagen wurden mit E. coli C 600 Hfl auf NZYDT-Platten ausplattiert und über Nacht bei 37°C inkubiert. Die so erhaltene cDNA-Bibliothek enthielt 2x10⁶ unabhängige Klone. Nach Amplifikation der cDNA-Bibliothek entsprechend herkömmlicher Methoden wurden 500 000 Phagen mit C 600 Hfl Zellen ausplattiert. Die Phagen wurden auf Nitrocellulose-Filter übertragen, mit 0,5 N NaOH/1,5 M NaCl lysiert und die denaturierte DNA durch 2-stündiges Backen bei 80°C fest an das Filter gebunden. Die Filter wurden in 6 x SET-Puffer (1 x SET = 0,15 M NaCl, 15 mM Tris/HCl, pH 7,4, 1 mM EDTA), 0,1 % SDS und 5 x Denhardt's Lösung (100 x Denhardt = 1 g Ficoll, 1 g Polyvinylpyrrolidon, 1 g BSA pro 50 ml) für 4 h, bei 68°C vorhybridisiert.

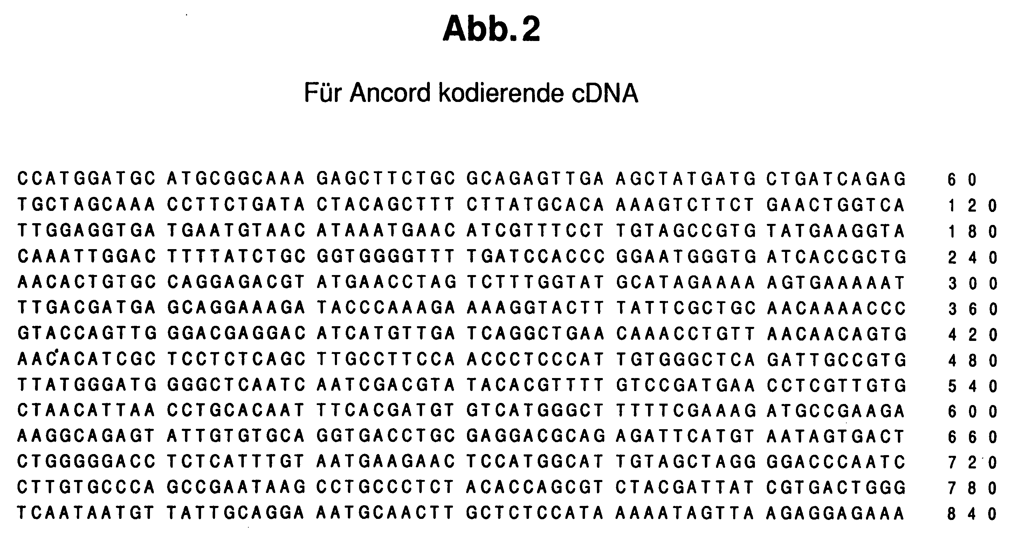

Hybridisiert wurde mit einer Nick-translatierten cDNA (Abb. 2), welche für Ancrod-Protein kodiert.

Die Filter wurden in einer Lösung, die 2 x SET, 0,1 % SDS, 30 % Formamid, 5 x Denhardt's und 10 % Dextransulfat enthielt, über Nacht bei 42°C unter leichtem Schütteln inkubiert. Danach wurden sie mehrfach in 2 x SET/0,1 % SDS bei 42°C gewaschen, angetrocknet und einem Röntgenfilm exponiert. Klone, die beim "Screening" eine radioaktive Antwort gaben, wurden isoliert und weitergezüchtet, um die entsprechende Phagen-DNA zu gewinnen.

Phagen-DNA wurde durch Inkubation der gereinigten Phagen mit Protenase K (ad 60 »g/ml) bei 55°C für 1 h und anschließender Phenol/Chloroformextraktion präpariert. Nach Zugabe von 3 Volumen Ethanol (-20°C) fiel die Phagen-DNA aus und wurde mit einer sterilen Injektionsnadel in 70 %iges Ethanol überführt, gewaschen und kurz sedimentiert. Nach kurzem Trocknen des Pellets an der Luft wurde es in TE-Puffer suspendiert.

Die gereinigte Phagen-DNA wurde auf Nitrozellulosefilter übertragen, renaturiert, reneutralisiert, gebacken und vorhybridisiert wie oben beschrieben. Hybridisiert wurde danach unter stringenten Bedingungen, mit einer radioaktiv markierten Oligonukleotidprobe, welche zur Ancrod-kodierten cDNA homolog war:
5' GTC TAC GAT TAT CGT GAC TGG GTC AA 3'
Die Filter wurden in einer Lösung, die 2 x SET, 0,1 % SDS, 30 % Formamid, 5 x Denhardt's und 10 % Dextransulfat enthielt, über Nacht bei 42°C unter leichtem Schütteln inkubiert. Danach wurden sie mehrfach in 2 x SET/0,1 % SDS bei 60°C gewaschen, angetrocknet und einem Röntgenfilm exponiert.

Die DNA, welche unter diesen Bedingungen nicht hybridisierte, wurde zur weiteren Analyse in dem einzelsträngigen Phagen MB Subkoniert.

### Beispiel 2

### Herstellung von einzelsträngiger DNA, die für Ancrod kodiert

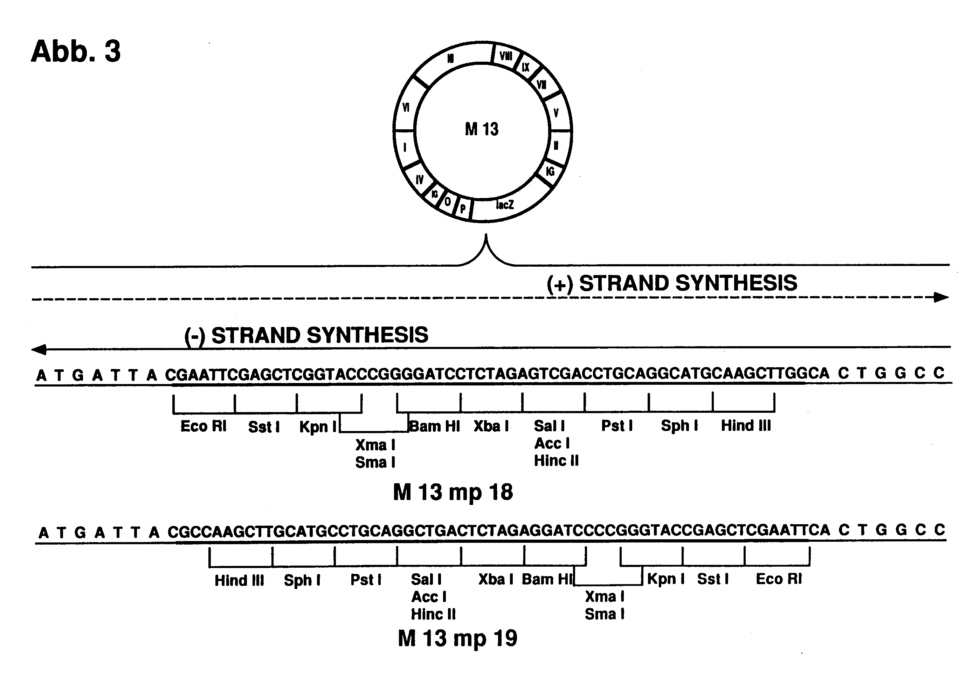

Ausgangspunkt waren die in Beispiel 1 beschriebene Phagen-DNA, welche mit dem Ancrod-spezifischen Oligonukleotid nicht hybridisierte. Sie wurden jeweils einzeln präparativ mit dem Restriktionsenzym Eco RI geschnitten. Die Eco RI-Fragmente, welche die cDNA-Inserts enthielten, wurden elektrophoretisch aus dem Gel eluiert. Jeweils 30 ng dieser Fragmente wurden bei 4°C für 12 h mit 100 ng des Eco RI geschnittenen, kommerziell erhältlichen Klonierungsvektors M13mp18 oder M13mp19 (Abb. 3) ligiert. Das Volumen des Ligationsansatzes betrug 10 »l. Die Ligation wurde durch 5 min Erhitzen auf 80°C beendet.

1/10 Volumen eines jeden Ligationsansatzes wurde zur Transformation von 100 »l kompetenten SR 101 Zellen eingesetzt. Nach Beendigung der Transformation wurden dem Transformationsansatz 60 »l 0,2 M IPTG-Lösung und 120 »l XGal (20 mg/ml) zugesetzt. Dieser Ansatz wurde in NZYDT-Topagar auf NZYDT-Agarplatten mit 200 »l SR 101 Zellen (OD₆₀₀=1) ausplattiert. Das Medium NZYDT ist kommerziell erhältlich (GIBCO-BRL). Klone, welche cDNA-Inserts enthielten, konnten aufgrund fehlender Blaufärbung der Plaques identifiziert werden. Mittels DNA-Sequenzanalyse (Sanger et al., Proc. Natl. Acad. Sci. USA 74, 1977, 5463-67) wurde die DNA-Sequenz dieses cDNA-Inserts aufgeklärt (Sequenzprotokoll, Sequenzen Nr. 6 bis 9).

### Beispiel 3

### Konstruktion von Vektoren für die Expression von Ancrod in eukaryontischen Zellen

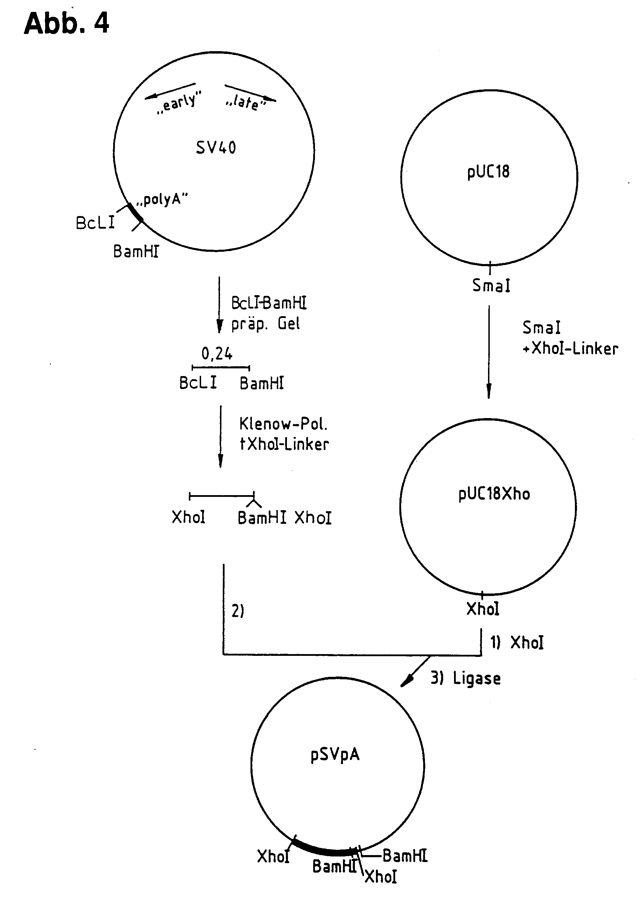

DNA des Affenvirus SV40 wurde mit den Restriktionsenzymen BamHI und BclI geschnitten und das 0,24 kb-Fragment gelelektrophoretisch präpariert (Abb. 4). Die Enden wurden in Gegenwart der vier Desoxynukleotidtriphosphate dATP, dCTP, dGTP und dTTP mit Klenow-Fragment aufgefüllt. Anschließend wurden XhoI-Linker anligiert.

Parallel wurde der kommerziell erhältlich Vektor pUC18 mit dem Enzym SmaI linearisiert. Dann wurden ebenfalls XhoI-Linker angesetzt. DNA dieses Vektors ("pUC18Xho") wurde mit XhoI linearisiert, mit alkalischer Phosphatase behandelt und mit dem 0,24 kb XholI-SV40-Fragment (s.o.) ligiert. Es entstand pSVpA.

pSVpA-DNA wurde präparativ mit XhoI gespalten und wie oben mit Klenow-Polymerase in Gegenwart der vier dNTPs inkubiert. Das 0,24 kb-Fragment wurde Gel-isoliert.

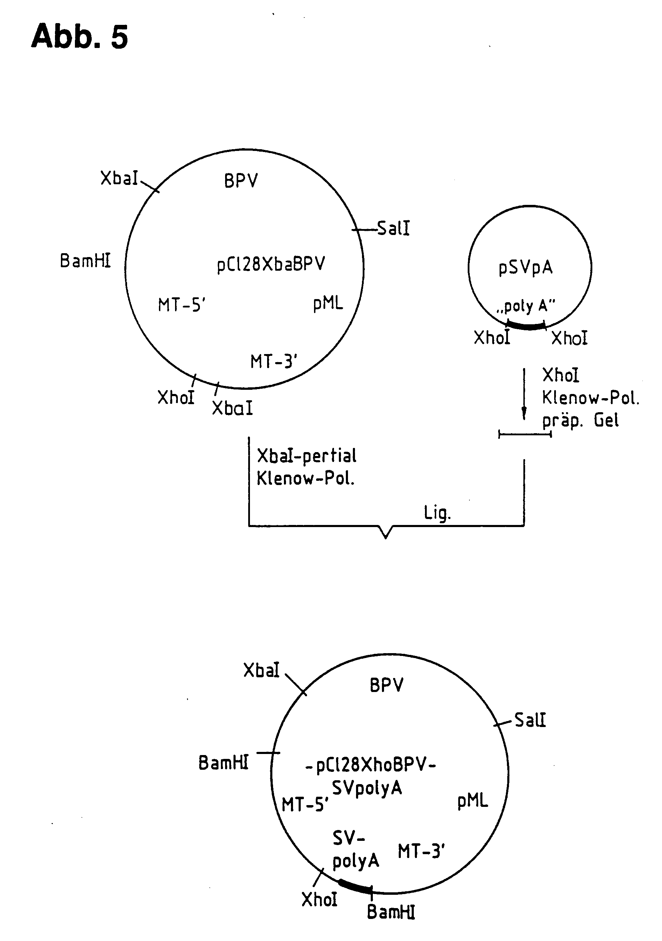

Gleichzeitig wurde der Eukaryonten-Expressionsvektor CL28XhoBPV, entstanden durch Ligation von CL28x und pB2-2 (nach Reddy et al. DNA 6, 1987, 461-72), partiell mit dem Restriktionsenzym XbaI geschnitten, d.h. es wurde zeitlich derart limitiert inkubiert, daß Moleküle entstanden, die nur an einer der beiden XbaI-Erkennungssequenzen gespalten, also linearisiert sind (Abb. 5). Der Ansatz wurde dann wie beschrieben mit Klenow-Polymerase und dNTPs umgesetzt. Die linearen Moleküle wurden anschließend durch Gelelektrophorese isoliert.

Es erfolgte dann die Ligation der linearen pCL28XhoBPV-Fragmente mit dem vorbehandelten 0,24 kb-Fragment aus SV40. Nach Transformation und Screening von Minilysaten wurde ein Klon isoliert, der das SV40-Fragment in der etwa 0,15 kB 3'-wärts der XhoI-Stelle gelegenen vormaligen XbaI-Stelle trug; diese DNA ("pCL28XhoBPV-SVpoly-A") trug die SV40-Transkriptionsstopsignale der "frühen" Gene.

Plasmid-DNA von pCL28XhoBPV-SVpolyA wurde mit dem Restriktionsenzym XhoI linearisiert und mit alkalischer Phosphatase behandelt. Gleichzeitig wurden die im Beispiel 2 beschriebenen cDNA-Inserts, welche nicht mit dem Ancrod-spezifischen Oligonukleotid hybridisierten, mit Xho-Linker mittels T4 Ligase versehen. Beide Fragmente wurden mittels T4-Ligase miteinander verbunden. Nach Transformation und Anlayse von Minilysaten wurde ein Klon isoliert, der die cDNA-Inserts einfach und in der korrekten Orientierung enthielt: pCL28BPV-Fibrogenase I-IV.

### Beispiel 4

### Transfektion und Etablierung von Zellinien

c127I Zellen (J. Virol. 26 (1978) 292; ATCC catalogue of cell lines and hybridomas 5th edition, 1985, p142) wurden mit BPV-Expressionsplasmiden transfiziert mit der Calciumphosphat-Copräzipitationsmethode (Virology 52 (1973), S. 456, DNA cloning; volume II, ed. D.M. Glover IRL Press, (1985) Seiten 143ff und 213).

5 x 10⁵ C127I-Zellen wurden in DMEM (Dulbeccos's Modified Eagles Medium) + 10 % FCS (Foetales Kalbsserum) in 60 mm Petrischalen eingesät. Am nächsten Tag wurde das Medium gewechselt auf MEM (Modified Eagles Medium) mit 25 nM Hepes + 10 % FCS. mit 10⁻⁵ g CsCl-gereinigter Plasmid DNA wurde ein Ca-Phosphat-Copräzipitat gebildet, welches vorsichtig auf die C127I-Zellen aufgebracht wurde. Die Zellen wurden 4 h bei 37°C; 7 % CO₂ inkubiert. Durch eine anschließende Glycerin-Schock-Behandlung wurde die Effizienz der Transfektion erheblich gesteigert. Hierzu wurde 4 h nach Aufbringen des Präzipitates das Medium von den Zellen abgezogen. Die Zellen wurden 3 min mit je 2 ml 15 % Glycerin/HBS (DNA cloning Vol. II, Seite 152) in einer 60 mm Petrischale bei Raumtemperatur inkubiert. Die Glycerin/HBS-Lösung wurde abgezogen und der Zellrasen mit 3 ml DMEM + 10 % FCS gewaschen. Die Zellen wurden mit DMEM + 10 % FCS bei 37°C; 7 % CO₂ inkubiert. Dreimal in der Woche wurde das DMEM + 10 % FCS abgezogen und durch frisches ersetzt. Nach 2 - 3 Wochen waren transfizierte Zellen, die das BPV-Genom enthalten, als Ansammlungen transformierter Zellen, sogenannte Foci, zu erkennen.

Nach Subklonierung der beschriebenen Foci wurden die Mediumüberstände der einzelnen Subklone nach bekannten Methoden auf Fibrinogen-spaltenden Aktivität untersucht.

Zur Produktion wurden die Zellinien nach Erreichen der Konfluenz in serumfreiem DMEM-Medium gehalten. Die neuen Fibrinogenasen können aus dem so erhaltenen serumfreien Zellkulturüberstand nach herkömmlichen proteinchemischen Methoden aufgereinigt und für pharmakologische und proteinchemische Analysen verwendet werden.

### Beispiel 5

### Isolierung und Reinigung eines fibrinogenspaltenden Enzyms (Sequenzprotokoll, Sequenz Nr. 5) aus dem Schlangengift der Agkistrodon rhodostoma

550 mg Rohgift (Trockensubstanz) von Agkistrodon rhodostoma wurden in 20 ml 20 mM Na₂HPO₄, 0,01 % Tween® 80, 500 mM NaCl, pH 7,0 Puffer (= Puffer A) aufgenommen.
a) Chromatographie an Matrex Red A-Sepharose®:
   Eine Chromatographiesäule (⌀2,5 cm, l = 5,1 cm) wurde mit 25 ml Matrex Red A-Sepharose® (Fa. Amicon) gefüllt. Die Säule wurde mit 100 ml Puffer A äquilibriert und anschließend mit dem gelösten Rohgift beladen. Die Säule wurde mit 45 ml Puffer A nachgewaschen (Fluß: 120 ml/h) und anschließend mit 85 ml eines Puffers B, der aus 20 mM Na₂HPO₄ 2 M NaCl, 0,01 % Tween, pH 7,0 bestand, eluiert. Die UV-aktive Fraktion (280 nm) wurde gesammelt.
   Das Eluat wurde zweimal gegen 2,5 l 20 mM Na₂HPO₄, 0,01 % Tween® 80, pH 7,0 Puffer (= Puffer C) nacheinander in einem Dialyseschlauch (Visking Size 8,32/32) je 2 h dialysiert. Die Leitfähigkeit der dialysierten Rohre betrug ca. 2,2 mS/cm (4°C).
b) Chromatographie an Arginin-Sepharose®:
   Eine Chromatographiesäule (⌀ = 2,5 cm, l = 10 cm) wurde mit Arginin-Sepharose® (Fa. Pharmacia) gefüllt und mit 200 ml Puffer C äquilibriert.
   Das dialysierte Eluat (Vol. ca. 140 ml) der Matrex Red A-Sepharose® wurde mit einem Fluß von 120 ml/h auf die Säule gegeben.
   Der Durchlauf der Säule (ca. 180 ml) wurde gesammelt und weiterverarbeitet. An die Säule gebunden blieb u.a. Ancrod, das durch Elution mit Argininsalzen gewonnen werden kann.
c) Chromatofocussierung
   Eine Chromatographiesäule (⌀ = 0,5 cm, l = 5 cm) wurde mit 1 ml Gelmaterial PBE® 94® (Fa. Pharmacia) gefüllt. Die Säule wurde mit 5 Säulenvolumina 20 mM Tris/HCl, 0,01 % Tween® 80, pH 8,0 Puffer (= Puffer D) äquilibriert.
   Die Säule wurde mit 20 ml des Durchlaufs der Arginin-Sepharose® geladen.
   Zur Chromatographie wurde ein Gradient von Puffer D nach 20 mM Essigsäure/HCl, 0,01 % Tween® 80, pH 2,0 Puffer (= Puffer E) innerhalb von 25 min linear bei einem Fluß von 1 ml/min gefahren. Nach dieser Zeit wurde die Säule weitere 13 min mit Puffer E gefahren. Die hierbei eluierende UV-aktive Fraktion (280 nm) wurde gesammelt. Man erhielt ca. 1 ml einer Proteinlösung, die nach der Proteinbestimmung (Methode: s. Anal. Biochem. 153, 267-271) ca. 0,04 mg/ml enthielt.
d) Charakterisierung der gereinigten Fibrinogenase V
   d1) SDS-Gelelektrophorese
      Im Vergleich mit Standardproteinen zeigte die Proteinlösung eine Hauptbande (ca. 70 bis 90 %) bei ∼ 42000 Dalton.
   d2) N-terminale Sequenzierung
      Die N-terminale Sequenz der gereinigten Proteinlösung wurde bestimmt (vgl. Sequenzprotokoll, Sequenz Nr. 5).
   d3) Fibrinogenase-assay
      Zur Bestimmung von Fibrinogenase-Aktivitäten wurde Fibrinogen von dem zu testenden Enzym zu Des AA-Fibrinogen umgesetzt.
      Diese Reaktion ging einher mit einer Trübungszunahme, die photometrisch (DD 340 nm) verfolgt wurde.
      Die Quantifizierung der Aktivität erfolgte durch Eichung mit einem Ancrod-Standard (Arwin®) 3000 U/mg.
      Die Fibrinogenase-Aktivität des gereinigten Enzyms betrug etwa 500 U/mg.

Sequenzprotokoll

Sequenz Nr. 1: Aminosäuresequenz aus 234 Aminosäuren

Val Ile Gly Gly Asp Glu Cys Asn Ile Asn Glu His Arg Phe Leu Val
                5                   10                  15

Ala Leu Tyr Asp Ser Thr Thr Arg Asn Phe Leu Cys Gly Gly Val Leu
            20                  25                  30

Ile His Pro Glu Trp Val Ile Thr Ala Lys His Cys Asn Lys Lys Ser
        35                  40                  45

Met Val Leu Tyr Leu Gly Lys His Lys Gln Ser Val Lys Phe Asp Asp
    50                  55                  60

Glu Gln Glu Arg Phe Pro Lys Glu Lys His Phe Ile Arg Cys Asn Lys
65                  70                  75                  80

Pro Arg Thr Arg Trp Gly Glu Asp Ile Met Leu Ile Arg Leu Asn Lys
                85                  90                  95

Pro Val Xaa Asn Ser Glu His Ile Ala Pro Leu Ser Leu Pro Ser Gly
            100                 105                 110

Pro Pro Ile Val Gly Ser Val Cys Arg Val Met Gly Trp Gly Ser Ile
        115                 120                 125

Asn Lys Tyr Ile Asp Val Leu Pro Asp Glu Pro Arg Cys Ala Asn Ile
    130                 135                 140

Asn Leu Tyr Xaa Tyr Thr Val Cys Arg Gly Val Phe Pro Arg Ile Gly
145                 150                 155                 160

Lys Lys Ser Lys Ile Leu Cys Ala Gly Asp Leu Gln Gly Arg Leu Asp
                165                 170                 175

Ser Cys His Cys Asp Ser Gly Gly Pro Leu Ile Cys Ser Glu Glu Phe
            180                 185                 190

His Gly Ile Val Tyr Arg Gly Pro Asn Pro Cys Ala Gln Pro Asp Lys
        195                 200                 205

Pro Ala Leu Tyr Thr Asn Ile Phe Asp His Leu His Trp Ile Leu Ser
    210                 215                 220

Ile Val Ala Gly Xaa Ala Thr Cys Tyr Pro
225                 230

Sequenzprotokoll
Sequenz Nr. 2: Aminosäuresequenz aus 236 Aminosäuren

Val Val Gly Gly Asp Glu Cys Asn Ile Asn Glu His Arg Phe Leu Ala
                5                   10                  15

Leu Val Tyr Ile Thr Ser Gly Phe Leu Cys Gly Gly Thr Leu Xab His
            20                  25                  30

Pro Glu Trp Val Val Ser Ala Ala His Cys Ala Arg Gly Glu Ile Glu
        35                  40                  45

Val Phe Phe Gly Val His Ser Leu Lys Asp Ile Arg Thr Asn Lys Asp
    50                  55                  60

Val Gln Lys Arg Val Ala Lys Glu Met Phe Phe Cys Leu Ser Ser Lys
65                  70                  75                  80

Xaa Tyr Thr Lys Trp Asp Lys Asp Ile Met Leu Ile Lys Leu Asp Ser
                85                  90                  95

Pro Val Xaa Asn Ser Thr His Ile Ala Pro Ile Ser Leu Pro Ser Ser
            100                 105                 110

Pro Pro Ser Val Gly Ser Val Cys Arg Val Met Gly Trp Gly Val Thr
        115                 120                 125

Thr Ser Pro Xaa Gly Thr Xab Pro Ser Val Pro His Cys Ala Asn Ile
    130                 135                 140

Asn Ile Leu Asp Tyr Xab Val Cys Arg Ala Ala Arg Pro Lys Leu Pro
145                 150                 155                 160

Ala Lys Ser Arg Thr Leu Cys Ala Gly Ile Leu Glu Gly Gly Lys Ser
                165                 170                 175

Ala Cys Asp Gly Asp Ser Gly Gly Pro Leu Asn Cys Asn Gly Glu Ile
            180                 185                 190

Gln Gly Ile Val Ser Trp Gly Gly Asn Ile Cys Ala Gln Pro Arg Lys
        195                 200                 205

Pro Ala His Tyr Xab Lys Val Ala Asp Tyr Thr Asp Trp Ile Lys Ser
    210                 215                 220

Ile Ile Ala Gly Xaa Thr Thr Ala Thr Cys Pro Pro
225                 230                 235

Sequenzprotokoll
Sequenz Nr. 3: Aminosäuresequenz aus 236 Aminosäuren

Val Ile Gly Gly Ala Glu Cys Asn Val Asn Glu His Arg Phe Leu Val
                5                   10                  15

Ala Leu Tyr Asp Xaa Leu Thr Gly Thr Leu Gln Cys Gly Gly Thr Leu
            20                  25                  30

Ile His Pro Glu Trp Val Leu Thr Ala Ala His Cys Asp Arg Lys Ser
        35                  40                  45

Met Val Ile Tyr Leu Gly Met His Xaa Lys Ser Val Asn Asn Asp Asp
    50                  55                  60

Gln Gln Arg Arg Ser Ala Lys Glu Lys Tyr Phe Phe Ser Cys Ser Lys
65                  70                  75                  80

Ser Ile Ala Ala Trp Glu Lys Asp Ile Met Leu Ile Arg Leu Asp Ser
                85                  90                  95

Pro Val Xaa Asn Ser Thr His Ile Ala Pro Leu Ser Leu Pro Ser Arg
            100                 105                 110

Pro Pro Thr Val Gly Ser Val Cys Arg Val Met Gly Trp Gly Ala Ile
        115                 120                 125

Thr Ser Pro Lys Glu Thr Tyr Pro Glu Val Pro His Cys Thr Asp Ile
    130                 135                 140

Asn Leu Leu Xaa Tyr Ser Glu Cys His Gly Asp Phe Pro Arg Leu Arg
145                 150                 155                 160

Ala Thr Ser Arg Ile Leu Cys Ala Gly Val Leu Gln Gly Gly Ile Asp
                165                 170                 175

Thr Cys Asn His Asp Ser Gly Gly Pro Leu Ile Cys Asp Glu Gln Phe
            180                 185                 190

Gln Gly Ile Val Ser Trp Gly Pro Tyr Pro Cys Ala Gln Pro Arg Asn
        195                 200                 205

Ala Ala Ile Tyr Thr Lys Val Phe Asn Tyr Leu Val Trp Val Trp Ser
    210                 215                 220

Thr Ile Ala Gly Xaa Thr Thr Val Thr Cys Pro Pro
225                 230                 235

Sequenzprotokoll
Sequenz Nr. 4: Aminosäuresequenz aus 234 Aminosäuren

Val Val Gly Gly Asn Glu Cys Asn Ile Asn Glu His Arg Phe Leu Val
                5                   10                  15

Ala Ile Phe Xab Ser Thr Gly Phe Val Cys Ala Gly Thr Leu Ile His
20 25 30

Pro Glu Trp Val Val Thr Ala Ala His Cys Glu Ser Thr Asp Leu Lys
35 40 45

Met Lys Phe Gly Met His Ser Lys Lys Val Gln Asn Glu Asp Glu Gln
50 55 60

Thr Arg Asn Ala Lys Glu Lys Phe Ile Cys Pro Asn Lys Lys Asn Asp
65 70 75 80

Glu Val Leu Asp Lys Asp Ile Met Leu Ile Lys Leu Asn His Pro Val
85 90 95

Ser Asn Ser Glu His Ile Ala Pro Leu Ser Leu Pro Ser Ser Pro Pro
100 105 110

Ser Val Gly Ser Phe Cys His Ile Met Gly Trp Gly Ser Ile Thr Pro
115 120 125

Val Lys Val Thr Phe Pro Asp Val Pro His Cys Ala Asn Ile Asn Leu
130 135 140

Leu Glu Glu Ala Glu Cys His Ala Gly Tyr Pro Glu Val Leu Ala Glu
145 150 155 160

Tyr Arg Thr Leu Cys Ala Gly Ile Val Gln Gly Gly Lys Asp Thr Cys
165 170 175

Met Tyr Asp Ser Gly Gly Pro Leu Ile Cys Asn Glu Gln Val Gln Gly
180 185 190

Ile Val Ser Tyr Gly Ala His Pro Cys Gly Gln Pro Leu Lys Pro Gly
195 200 205

Ile Tyr Thr Arg Leu His Asp Tyr Asn Asp Trp Ile Asn Ser Ile Met
210 215 220

Ala Gly Asn Thr Ala Val Thr Cys Pro Pro
225 230

Sequenzprotokoll

Sequenz Nr. 5: Aminosäuresequenz aus 25 Aminosäuren

Val Ile Gly Gly Asp Glu Cys Asn Ile Asn Glu His Pro Phe Leu Val
                5                   10                  15
Ala Val Tyr Glu Glu Thr Ala Gly Ala
            20                  25

Sequenzprotokoll

Sequenz Nr. 6: Nukleotidsequenz aus 1096 Nukleotiden, korrespondiert
               mit der Aminosäuresequenz niedergelegt in Sequenz Nr.1

Strangform: Doppelstrang
Topologie: Linear
Art des Moleküls: cDNA zur mRNA
Ursprüngliche Herkunft: Agkistrodon rhodostoma
Die für das in Sequenz Nr. 1 beschriebene Protein kodierende Region
beginnt mit Base 144 und endet mit Base 841.

GAATTCCATG GATGCATGCG TTTGGGACTG GGATCTTACA GGCAAAGAGC TTTCTGTGCA 60
GAGTTGAAGC TATGGTGCTG ATCAGAGTGC TAGCAAACCT TGTGATACTA CAGCTTTCTT 120
ACGCACAAAA GTCTTCTGAA CTGGTCATTG GAGGTGATGA ATGTAACATA AATGAACATC 180
GTTTCCTTGT AGCCTTGTAT GACAGTACGA CTCGGAATTT TCTCTGTGGT GGGGTTTTGA 240
TCCATCCGGA ATGGGTGATC ACTGCTAAAC ACTGCAACAA GAAAAGTATG GTCCTATACC 300
TTGGTAAGCA TAAACAAAGT GTAAAATTTG ACGATGAGCA GGAAAGATTC CCAAAGGAGA 360
AGCACTTTAT TCGCTGTAAC AAACCCCGTA CCAGATGGGG CGAGGACATC ATGTTGATCA 420
GGCTGAACAA ACCTGTTAAC AACAGTGAAC ACATCGCTCC TCTCAGCTTG CCTTCCGGCC 480
CTCCCATTGT GGGCTCAGTT TGCCGTGTTA TGGGATGGGG CTCAATCAAT AAATATATAG 540
ACGTTTTGCC CGATGAACCT CGTTGTGCTA ATATTAACCT GTACAATTAC ACGGTGTGTC 600
GTGGAGTTTT TCCAAGGATA GGAAAGAAAA GCAAAATATT GTGTGCAGGT GACCTGCAAG 660
GACGCCTAGA TTCATGTCAC TGTGACTCTG GGGGACCTCT CATTTGTAGT GAAGAATTCC 720
ATGGCATTGT ATATCGGGGA CCCAATCCTT GTGCCCAACC AGATAAGCCT GCCCTCTACA 780
CCAACATCTT CGATCATCTT CACTGGATCC TTAGCATTGT GGCAGGAAAT GCAACTTGCT 840
ATCCATAAAA CCTTTTGAAA TAGTTAAGTG GAGAAAATGT AACATATTAG TAAATCTCTT 900
CTATATCCTT GCATTGGAAC ATATTCCCAG GCTGTAAGCT TTTTAGACTC AAATAGGACT 960
ACCTTTGGAG TAAGAAGTGC TCAAAATAGT GCTGCAGGGA TCATGTCCCA TTTAATTTCA 1020
GTTTAAAACA GTCTCCATAG ATTGGAGGCC TGTTTAGGGT TAGGTGCAAA TTTCTGACTC 1080
TAAATGGACC ATTCCC

Sequenzprotokoll

Sequenz Nr. 7: Nukleotidsequenz aus 1333 Nukleotiden, korrespondiert mit
der Aminosäuresequenz niedergelegt in Sequenz Nr. 2

Strangform: Doppelstrang
Topologie: Linear
Art des Moleküls: cDNA zu mRNA
Ursprüngliche Herkunft: Agkistrodon rhodostoma
Die für das in Sequenz Nr. 2 beschriebene Protein kodierende Region
beginnt mit Base 231 und endet mit Base 935.

ANCCCCCTTT NNNGGNGGGG GGGGNCCAGA AGTTNCCCAG ATTNCTTGGC CACCCCGGTT 60
GCTTAATTTG ATCAAATAAA GTGCTGCTTG ATCCAAGAAA TTCTCCGCTT GGGTTATCTG 120
ATTAGGCAAA CAGCTTGCCA CGCAGAGTTG AAGCTATGGT GCTGATCAGA GTGCTAGCAA 180
ACCTTCTGAT ACTACAACTT TCTNACGCAC AAAAGTCATC TGAACNGGNC GTTGGAGGTG 240
ATGAATGTAA CATAAATGAA CATCGTTTCC TTGCACTCGT GTATATCACT AGTGGTTTTC 300
TCTGCGGTGG GACTTTGANC CACCCGGAAT GGGTGGTCAG TGCTGCACAT TGCGCTAGGG 360
GAGAAATAGA GGTATTCTTT GGTGTGCATA GCCTAAAGGA TATACGGACA AATAAGGATG 420
TGCAGAAAAG AGTCGCAAAG GAGATGTTCT TTTGCCTCAG TAGCAAAAAC TATACCAAAT 480
GGGACAAGGA CATCATGTTA ATCAAGCTGG ACAGTCCTGT TAACAACAGT ACTCACATCG 540
CGCCTATCAG CTTGCCTTCC AGCCCTCCCA GTGTGGGCTC AGTTTGCCGT GTTATGGGAT 600
GGGGCGTAAC CACATCTCCT AATGGGACTA TNCCCAGTGT NCCTCACTGT GCTAACATTA 660
ACATACTCGA TTATNCGGTG TGTCGAGCAG CTAGGCCAAA GTTGCCGGCG AAAAGCAGAA 720
CATTATGTGC TGGTATCCTG GAAGGAGGCA AAAGTGCATG TGACGGTGAC TCTGGGGGAC 780
CCCTCAACTG TAATGGAGAA ATCCAGGGCA TTGTATCTTG GGGGGGTAAT ATTTGTGCTC 840
AACCGCGTAA GCCTGCCCAC TACNCCAAGG TCGCCGATTA TACTGATTGG ATTAAGAGCA 900
TTATTGCAGG AAATACAACT GCAACTTGCC CCCCGTGAAA ATTTTTGAAA AACTTAAGAG 960
GAGAAAATAC ATCTCTTCTA TATCCCTAGC CATATTCAAT TACATTGGAA TATATTCCCA 1020
AGTTAACTCT ACATCAACAA AAAATCCTAC NAAACAACAA CAGAGAAGGA GCAGATAAAA 1080
GAGATAAATG GTACAAAATT GAGAATCAAG ACTTAAAGAT GGAACTTAAG AAAACAAGGA 1140
ACCATGATTT AATCCTTGTG GGGGGGGAAA TCACAAGAAT TGGAAAAAAA CAACTTATCC 1200
CTTAGACAGC AAACTAAATC TGAGGACAAG AAAACAGATT GGATAAAATG GACTGTAGAA 1260
ATGTCAGGAA CATCGGAGAG AAAGGAAATA ATAAGAGAAG CAAAAAAAAA AAAAGCATGC 1320
ATCCATGGAA TTC

Sequenzprotokoll

Sequenz Nr. 8: Nukleotidsequenz aus 988 Nukleotiden, korrespondiert
               mit der Aminosäuresequenz niedergelegt in Sequenz Nr. 3

Strangform: Doppelstrang
Topologie: Linear
Art des Moleküls: cDNA zur mRNA
Ursprüngliche Herkunft: Agkistrodon rhodostoma
Die für das in Sequenz Nr. 3 beschriebene Protein kodierende Region
beginnt mit Base 197 und endet mit Base 904

AACAATAAAG NCTGCNTGAN CAAGAAGCNN CTGCTTAGCT TATCTGATAA GATTGACATG 60
TATCTCAAGC TTAAGTTGGG ACTGGGATCT TACAGCAAAG AGCTTTCCAC GCAGAGTTGA 120
AGCTATGGTG CTGATCAGAG TGCTAGCAAA CCTTCTGATA CTACAGCTTT CTTACGCACA 180
AAAGTCTTCT GAACTGGTCA TTGGAGGTGC TGAATGTAAC GTAAATGAAC ATCGTTTCCT 240
TGTAGCCTTG TATGACAATT TGACTGGGAC TTTGCAGTGT GGTGGGACTT TGATCCACCC 300
GGAATGGGTG CTCACTGCTG CGCACTGCGA CAGGAAAAGT ATGGTCATAT ACCTTGGTAT 360
GCATAACAAA AGTGTAAACA ATGACGATCA GCAGAGAAGA TCCGCAAAGG AGAAGTACTT 420
TTTTAGCTGT AGCAAAAGCA TTGCCGCATG GGAAAAGGAC ATCATGTTGA TCAGGCTGGA 480
CAGTCCTGTT AACAACAGTA CACACATCGC CCCTCTCAGC TTGCCTTCCA GACCTCCCAC 540
TGTGGGCTCA GTTTGCCGTG TTATGGGATG GGGCGCAATC ACATCTCCTA AAGAGACTTA 600
TCCTGAGGTC CCTCATTGTA CTGACATTAA CCTGTTAAAT TATTCGGAGT GTCATGGAGA 660
TTTCCCACGG TTGCGGGCGA CAAGCAGAAT ATTGTGTGCA GGTGTCCTGC AAGGAGGCAT 720
AGATACATGT AATCATGACT CTGGGGGACC TCTCATCTGT GATGAACAAT TCCAGGGCAT 780
TGTATCTTGG GGACCCTATC CTTGTGCCCA ACCGCGTAAC GCTGCCATCT ACACCAAAGT 840
CTTCAATTAT CTTGTCTGGG TCTGGAGCAC TATTGCAGGA AATACAACTG TGACTTGCCC 900
CCCATGAAAA CATTTTTATT TCCACAAAGG AGTTTCCAAA GGAATTAAAA CTAAATAATG 960
TGGTAAAAAA AAAAAAAAAA AAAAAAAA

Sequenzprotokoll

Sequenz Nr. 9: Nukleotidsequenz aus 957 Nukleotiden, korrespondiert
               mit der Aminosäuresequenz niedergelegt in Sequenz Nr. 4

Strangform: Doppelstrang
Topologie: Linear
Art des Moleküls: cDNA zur mRNA
Ursprüngliche Herkunft: Agkistrodon rhodostoma
Die für das in Sequenz Nr. 4 beschriebene Protein kodierende Region
beginnt mit Base 210 und endet mit Base 911.

CTNAATTNNA CAAAAAAAGT GCTGCTTGGT CAAGAGGTNC TCCGCTTCGG TTATCTGATT   60
AGATTGATAC GTATCTCAAG TATAAGTTTG GGACTGGGAT CTTACAGGAA AACAGCTTTC   120
CGTGCAGAGT TGAAGTTATG GTACTGATCA GAGTGCTAGC AAACCTTCTG ATACTACAGC   180
TTTCTTACGC ACAAAAGTCA TCTGAACTGG TCGTTGGAGG TAATGAATGT AACATAAATG   240
AACATCGTTT CCTTGTAGCC ATCTTTAACT CTACTGGGTT TGTCTGCGCT GGGACTTTGA   300
TCCACCCAGA ATGGGTGGTC ACTGCTGCAC ACTGCGAGAG TACGGATCTC AAGATGAAGT   360
TTGGTATGCA TAGCAAAAAG GTACAAAATG AGGATGAGCA GACAAGAAAC GCAAAGGAAA   420
AGTTCATTTG TCCCAATAAG AAAAACGATG AAGTACTGGA CAAGGACATT ATGTTGATCA   480
AGCTGAACCA TCCTGTTAGC AATAGTGAAC ACATCGCGCC TCTCAGCTTG CCTTCCAGCC   540
CTCCCAGTGT GGGCTCATTT TGCCATATTA TGGGATGGGG CTCAATCACA CCTGTTAAAG   600
TGACTTTCCC CGATGTCCCT CATTGTGCTA ACATTAACCT ACTCGATGAT GCAGAGTGTC   660
ATGCAGGTTA CCCTGAGGTG CTGGCAGAAT ACAGAACATT GTGTGCAGGT ATCGTGCAAG   720
GAGGCAAAGA TACATGTATG TATGACTCTG GAGGACCTCT CATCTGTAAT GAACAAGTCC   780
AGGGCATTGT ATCTTATGGG GCGCATCCTT GTGGCCAACC TCTTAAGCCT GGTATCTACA   840
CCAGGCTCCA TGATTATAAT GACTGGATCA ACAGCATTAT GGCAGGAAAT ACAGCTGTGA   900
CTTGCCCCCC GTGAAAACTT TAGTATCAGA AGGTTTGCTG CATGCATCCA TGAATTC

## Patentansprüche

1. Glykosylierte, partiell glykosylierte oder nicht glykosylierte Polypeptide mit den im Sequenzprotokoll angegebenen Aminosäuresequenzen 1, 2, 3 und 4, worin Xaa und Xab Reste natürlicher α-Aminosäuren darstellen, sowie deren Allelvarianten, die mit den angegebenen Sequenzen in mehr als 95 % der Aminosäurepositionen identisch sind.

2. Polypeptid, erhältlich aus dem Rohgift von Agkostrodon rhodostoma durch Chromatographie an Matrex Red A-Sepharose ^{(R)} und Arginin-Sepharose ^{(R)} sowie anschließende Chromatofocussierung mit dem Gelmaterial PBE^{(R)} 94, welches eine Hauptbande mit dem Molekulargewicht von etwa 42.000 Dalton aufweist, eine Fibrinogenase-Aktivität von ca. 500 U/mg zeigt und die N-terminale Aminosäuresequenz 5 besitzt.

3. DNA-Sequenzen, dadurch gekennzeichnet, daß sie für die Polypeptide gemäß Anspruch 1 oder 2 kodieren.

4. Rekombinantes DNA-Molekül, welches eine DNA-Sequenz nach Anspruch 3 enthält.

5. Rekombinantes DNA-Molekül nach Anspruch 4, das eine DNA-Sequenz nach Anspruch 3 enthält, welche funktionell mit einer Expressions-Kontrollsequenz verbunden ist, die die Expression in geeigneten Wirtssystemen ermöglicht.

6. Rekombinantes DNA-Molekül nach Anspruch 5, dadurch gekennzeichnet, daß die Expressions-Kontrollsequenz ein E.coli-Promotorsystem, ein Promotorsystem eines E.coli Bakteriophagen, eine Hefe- oder Pilz-Expressions-Kontrollsequenz oder eine andere eukaryontische Expressions-Kontrollsequenz ist.

7. Wirtsorganismus, dadurch gekennzeichnet, daß er mindestens ein rekombinantes DNA-Molekül gemäß Anspruch 4, 5 oder 6 enthält.

8. Wirtsorganismus nach Anspruch 7, dadurch gekennzeichnet, daß er ein Bakterium, eine Hefe, ein Pilz, eine tierische oder eine menschliche Zelle ist.

9. Gentechnisches Verfahren zur Herstellung der Polypeptide gemäß Anspruch 1, dadurch gekennzeichnet, daß man in einem geeigneten Wirtsorganismus DNA-Sequenzen zur Expression bringt, die für Aminosäuresequenzen nach Anspruch 1 kodieren.

10. Polypeptide gemäß Anspruch 1 oder 2 zur Verwendung bei der Vorbeugung und Bekämpfung von Krankheiten.

11. Verwendung eines Polypeptids gemäß Anspruch 1 oder 2 zur Herstellung von Arzneimitteln zur Behandlung und Prophylaxe thromboembolischer Erkrankungen und Glomerulonephritis.

## Claims

1. A glycosylated, partially glycosylated or non-glycosylated polypeptide with the amino-acid sequence 1, 2, 3 or 4 given in the list of sequences, where Xaa and Xab are residues of natural α-amino acids, and the allelic variants thereof which are identical in more than 95% of the amino-acid positions to the indicated sequences.

2. A polypeptide obtainable from the crude venom of Agkistroden rhodostoma by chromatography on Matrex red A-Sepharose® and arginine-Sepharose®, and subsequent chromatofocusing with the gel material PBE®94, which displays a main band with the molecular weight of about 42,000 Dalton, shows a fibrinogenase activity of about 500 V/g and has N-terminal amino-acid sequence 5.

3. A DNA sequence which codes for the polypeptide as claimed in claim 1 or 2.

4. A recombinant DNA molecule which contains a DNA sequence as claimed in claim 3.

5. A recombinant DNA molecule as claimed in claim 4, which contains a DNA sequence as claimed in claim 3, which is functionally connected to an expression control sequence which makes expression in suitable host systems possible.

6. A recombinant DNA molecule as claimed in claim 5, wherein the expression control sequence is an E.coli promoter system, a promoter system of an E.coli bacteriophage, a yeast or fungus expression control sequence or another eukaryotic expression control sequence.

7. A host organism which contains at least one recombinant DNA molecule as claimed in claim 4, 5 or 6.

8. A host organism as claimed in claim 7, which is a bacterium, a yeast, a fungus, or an animal or human cell.

9. A method of genetic manipulation for preparing a polypeptide as claimed in claim 1, which comprises bringing about the expression, in a suitable host organism, of a DNA sequence which codes for an amino-acid sequence as claimed in claim 1.

10. The use of a polypeptide as claimed in claim 1 or 2 for preventing and controlling diseases.

11. The use of a polypeptide as claimed in claim 1 or 2 for producing drugs for the treatment and prophylaxis of thromboembolic disorders and glomerulonephritis.

## Revendications

1. Polypeptides glycosylés, partiellement glycosylés, ou non glycosylés, avec les séquences d'acides aminés 1, 2, 3 et 4 indiquées dans le protocole de séquence où Xaa et Xab représentent des restes d'α acides aminés naturels ainsi que leur variantes allèles qui sont identiques aux séquences indiquées dans plus de 95 % des positions d'acides aminés.

2. Polypeptide obtenu du poison brut de Agkostrodon rhodostoma par chromatographie sur matrice Red-A Sepharose® et Arginine-Sepharose® ainsi qu'ensuite par chromatofocalisation avec la matière gel PBE ®94 qui comporte une bande principale du poids moléculaire d'environ 42000 Dalton, présente une activité fibrinogénase d'environ 500 U/mg et possède la séquence d'acides aminés N-terminale 5.

3. Séquence de DNA, caractérisée par le fait qu'elle code pour les polypeptides selon les revendications 1 ou 2.

4. Molécule de DNA recombinante qui contient une séquence de DNA selon la revendication 3.

5. Molécule de DNA recombinante selon la revendication 4 qui contient une séquence de DNA selon la revendication 3 qui est reliée fonctionnellement avec une séquence de contrôle d'expression permettant l'expression en système hôte approprié.

6. Molécule de DNA recombinante selon la revendication 5, caractérisée par le fait que la séquence de contrôle d'expression est un système promoteur de E-coli, un système promoteur d'une bactérie phage de E-coli, une séquence de contrôle d'expression de levure ou de champignon ou une autre séquence de contrôle d'expression eucaryontique.

7. Organisme hôte, caractérisé par le fait qu'il contient au moins une molécule de DNA recombinante selon la revendication 4, 5 ou 6.

8. Organisme hôte selon la revendication 7, caractérisé par le fait qu'il est une bactérie, une levure, un champignon, une cellule animale ou humaine.

9. Procédé de technique génétique pour la préparation de polypeptides selon la revendication 1, caractérisé par le fait que, dans un organisme hôte approprié, on porte à expression des séquences de DNA qui codent pour des séquences d'amino-acides selon la revendication 1.

10. Polypeptides selon la revendication 1 ou 2 pour utilisation à la prévention et la lutte contre les maladies.

11. Utilisation d'une polypeptide selon la revendication 1 ou 2 pour la préparation de médicaments pour le traitement et la prophylaxie des atteintes thromboembolitiques et des glomérulonéphrites.
